# EUROPEAN PATENT APPLICATION

(11) **EP 2 695 571 A1**
(43) Date of publication of application: **12.02.2014**
(21) Application number: 13179234.3
(22) Date of filing: 05.08.2013
(51) Int. Cl.: A61B 3/135

(54) **Slit lamp microscope**

(30) Priority: 08.08.2012 JP 2012176525
(71) Applicant: Kabushiki Kaisha Topcon, Tokyo 174-8580 (JP)
(72) Inventor: Nomura, Kazuhisa, Tokyo 174-8580 (JP); Watanabe, Takahiro, Tokyo 174-8580 (JP); Takeda, Takanori, Tokyo 174-8580 (JP)
(74) Representative: Vossius & Partner

(57) **Abstract**

A slit lamp microscope of an embodiment comprises a light source, pair of slit blades, actuator, mechanism, operation part, storage, operation-content detector and controller. The light source emits illumination light. The pair of slit blades generates slit light from the illumination light. The mechanism changes the interval between the slit blades based on a driving force generated by the actuator. The storage stores in advance correspondence information in which the operation content with respect to the operation part and the operation amount of the actuator are associated. The operation-content detector detects an operation content with respect to the operation part. The controller obtains the operation amount associated with the detected operation content based on the correspondence information and control the actuator based on the obtained operation amount, thereby changing the interval of the slit blades by a distance corresponding to this operation content.

## Description

### [Field of the Invention]

The present invention relates to a slit lamp microscope.

### [Background of the Invention]

A slit lamp microscope is an ophthalmologic apparatus that uses slit light to cut an optical section of a cornea, thereby obtaining an image of a cross-section of the cornea. The slit lamp microscope is provided with a pair of slit blades for obtaining the slit light. The user appropriately adjusts the interval between the slit blades (slit width) and conducts observation. This slit width adjustment requires high precision.

As slit lamp microscopes comprising a mechanism for changing the slit width, those described in Patent documents 1 to 3 are known. Disclosed in Patent document 1 is a slit lamp microscope in which enhancing the operability is attempted by automatically changing the amount of light from a light source in response to the slit width during slit width adjustment. In this slit lamp microscope, operations with respect to the slit width variable knobs installed on the left and right are mechanically conveyed, thereby changing the slit width.

Disclosed in Patent document 2 is a mechanism for changing the slit direction. Explained in greater detail, this slit lamp microscope is configured to rotate a rotation body installed with a slit board as well as an opening and closing mechanism thereof by rotating a slit direction conversion axis by operating a slit direction conversion lever, and conveying this rotation operation to a gear wheel mechanism. The change of the slit width is, in the same manner as Patent document 1, carried out by means of a mechanically conveying the operations with respect to the slit width variable knobs installed on the left and right.

The slit lamp microscope disclosed in Patent document 3 is configured such that the slit width is changed by means of converting the rotation operation with respect to a slit width adjusting handle to linear movement along a slit light projection optical axis and conveying this to two slit blades.

### [Prior Art Documents]

### [Patent Documents]

[Patent Document 1] Japanese Patent Publication S57-45569 (1982-45569)
[Patent Document 2] Japanese Published Utility Model Application S55-15521 (1980-155211)
[Patent Document 3] Japanese Published Unexamined Application S63-111835 (1988-111835)

### [Problem to be Solved by the Invention]

In this manner, slit width change is carried out upon receiving operations by the user. Meanwhile, as mentioned above, high precision is required for slit width adjustment. Moreover, the slit lamp microscope is frequently used in medical fields and is referred to as a "stethoscope for ophthalmologists". Accordingly, the user must frequently carry out subtle operations. This not only places a burden on the user but may also place a burden on the patients due to the prolongation of examinations.

This invention is developed in order to solve the problems mentioned above, with the objective of providing a slit lamp microscope capable of easily adjusting the slit width with high precision.

### [Means for Solving the Problem]

In order to achieve the aforementioned objects, the slit lamp microscope according to Claim 1 comprises: a light source configured to emit illumination light; a pair of slit blades configured to generate slit light from the illumination light; an actuator configured to generate a driving force; a mechanism configured to change the interval between the slit blades based on the driving force; an operation part; a storage configured to store in advance correspondence information in which the operation content with respect to the operation part and the operation amount of the actuator are associated; an operation-content detector configured to detect an operation content with respect to the operation part; and a controller configured to obtain the operation amount associated with the detected operation content based on the correspondence information and control the actuator based on the obtained operation amount, thereby changing the interval of the slit blades by a distance corresponding to this operation content.
The invention according to Claim 2 is the slit lamp microscope according to Claim 1, wherein the operation content includes the displacement of the operation part with respect to a predetermined standard operation position, and the displacement and the operation amount are associated in the correspondence information such that the operation amount changes in at least a part of a variable range of the displacement.
The invention according to Claim 3 is the slit lamp microscope according to Claim 2, wherein the standard operation position and the standard slit position of the slit blades such that the interval is substantially zero are associated in the correspondence information.
The invention according to Claim 4 is the slit lamp microscope according to Claim 3, comprising a slit detector configured to detect the state in which the slit blades are positioned in a predetermined detection position, wherein the storage stores in advance distance information indicating the distance between the standard slit position and the detection position, and when it is detected that the slit blades are positioned in the detection position, the controller controls the actuator based on the distance information, thereby positioning the slit blades in the standard slit position.
The invention according to Claim 5 is the slit lamp microscope according to any one from among Claims 2 to 4, wherein the operation part comprises a rotation operation part capable of rotating in a first direction and a second direction which is the opposite direction thereof, the operation content includes the rotation position of the rotation operation part with respect to the standard operation position, and the rotation position and the operation amount are associated in the correspondence information such that the amount of change of the operation amount with respect to the unit amount of change of the rotation position monotonically varies within a first range from the standard operation position towards the first direction.
The invention according to Claim 6 is the slit lamp microscope according to Claim 5, wherein, in the first range, the first direction is the rotation direction of the rotation operation part for increasing the interval of the slit blades, and the monotonic variation is a monotonic increase.
The invention according to Claim 7 is the slit lamp microscope according to Claim 5, wherein, in the first range, the first direction is the rotation direction of the rotation operation part for decreasing the interval of the slit blades, and the monotonic variation is a monotonic decrease.
The invention according to Claim 8 is the slit lamp microscope according any one from among Claims 5 to 7, wherein the rotation position and the operation amount are associated in the correspondence information such that, in a second range differing from the first range, the amount of change of the operation amount with respect to the unit amount of change of the rotation position in the first direction monotonically varies inversely with the monotonic variation.
The invention according to Claim 9 is the slit lamp microscope according any one from among Claims 5 to 8, wherein the rotation position and the operation amount are associated in the correspondence information such that it includes a third range in which zero is associated as the operation amount with respect to the change of the rotation position.
The invention according to Claim 10 is the slit lamp microscope according any one from among Claims 5 to 9, wherein the controller comprises a correspondence-information inverter configured to invert the correspondence between the rotation direction of the rotation operation part and the operation amount in the correspondence information.
The invention according to Claim 11 is the slit lamp microscope according any one from among Claims 1 to 10, wherein the storage stores in advance a plurality of the correspondence information, and the controller selectively uses the plurality of correspondence information to obtain the operation amount associated with the operation content detected by the operation-content detector.
The invention according to Claim 12 is the slit lamp microscope according any one from among Claims 1 to 11, wherein the actuator is a pulse motor, and the operation amount indicates the number of pulses of pulse signals transmitted from the controller to the pulse motor.

### [Effect of the Invention]

According to the slit lamp microscope related to the present invention, it is possible to easily adjust the slit width with high precision.

### [Brief Description of the Drawings]

Fig. 1 is a schematic side view illustrating an example of the exterior configuration of the slit lamp microscope related to the embodiment.
Fig. 2 is a schematic side view illustrating an example of a configuration of the optical system of the slit lamp microscope related to the embodiment.
Fig. 3 is a schematic block diagram illustrating an example of a configuration of the control system of the slit lamp microscope related to the embodiment.
Fig. 4A is a schematic diagram illustrating an example of the correspondence information of the slit lamp microscope related to the embodiment.
Fig. 4B is a schematic diagram illustrating an example of the correspondence information of the slit lamp microscope related to the embodiment.
Fig. 4C is a schematic diagram illustrating an example of the correspondence information of the slit lamp microscope related to the embodiment.
Fig. 5A is a schematic diagram for explaining the processes carried out by the slit lamp microscope related to the embodiment.
Fig. 5B is a schematic diagram for explaining the processes carried out by the slit lamp microscope related to the embodiment.
Fig. 6 is a flow chart illustrating an example of an operation of the slit lamp microscope related to the embodiment.

### [Mode for Carrying Out the Invention]

Examples of the embodiment of the slit lamp microscope related to the present invention are explained in detail with reference to the diagrams. It should be noted that contents of the document presented in this specification may be appropriately incorporated as contents of the embodiment below.

First, the directions are defined. The direction from the optical element positioned closest to the subject side in a device optical system is a "front direction", while the direction opposite from this is "rear direction". Moreover, the horizontal direction orthogonal to the front direction is a "left-right direction". Further, the direction orthogonal to both the front-rear direction and the left-right direction is a "vertical direction (upward-downward direction)".

### [Exterior configuration]

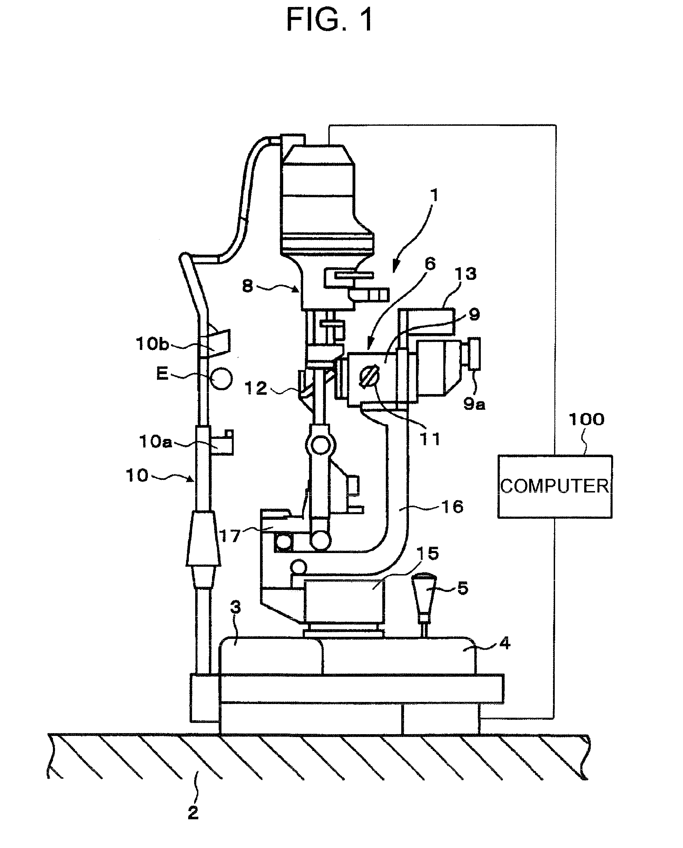

The exterior configuration of the slit lamp microscope related to the present embodiment is explained with reference to Fig. 1. A computer 100 is connected to the slit lamp microscope 1. The computer 100 carries out various control processes and arithmetic processes. Further, rather than providing a computer 100 separate from a main body of the microscope (a case for housing an optical system, etc.), a configuration is possible in which a similar computer is arranged within the main body of the microscope.

The slit lamp microscope 1 is placed on a table 2. It should be noted that the computer 100 may be arranged on the other table or other locations. A base 4 is configured to be movable in the horizontal direction via a moving mechanism 3. The base 4 moves by the tilting operation of an operation handle 5.

The top surface of the base 4 is provided with a supporting part 15 that supports an observation system 6 and an illumination system 8. A supporting arm 16 for supporting the observation system 6 is provided on the supporting part 15 such that it is capable of rotating in the left-right direction. A supporting arm 17 supporting the illumination system 8 is provided on the top part of the supporting arm 16 such that it is capable of rotating in the left-right direction. The supporting arms 16 and 17 are independently turnable around the same axis.

The observation system 6 moves by manually turning the supporting arm 16. The illumination system 8 moves by manually turning the supporting arm 17. It should be noted that the respective supporting arms 16 and 17 may be configured so as to be turned by an electric mechanism. In such cases, an actuator generating a driving force for turning the respective supporting arms 16 and 17 and a transmission mechanism transmitting this driving force are provided. The actuator is configured by, for example, a pulse motor. The transmission mechanism is configured by, for example, a combination of gearwheels, or rack and pinion, etc.

The illumination system 8 projects illumination light onto an eye E. The illumination system 8 may be, as mentioned above, swung in the left-right direction around a turning axis. Thereby, the projection direction of the illumination light with respect to the eye E changes. The illumination system 8 may be configured such that it is allowed to be swung vertically. That is, it may be configured such that the angle of elevation and the angle of depression of the illumination light can be changed.

The observation system 6 comprises a left-and-right pair of optical systems for guiding a returned light of the illumination light from the eye E. These optical systems are housed within a lens tube body 9. The end of the lens tube body 9 is an eyepiece 9a. The user observes the eye E by looking into the eyepiece 9a with the naked eyes. The lens tube body 9 may be turned in the left-right direction by means of turning the supporting arm 16 as mentioned above. Thereby, the direction of the observation system 6 with respect to the eye E may be changed. It should be noted that the returned light of the illumination light includes a variety of light components passing the eye E such as scattered light, and with this variety of light components collectively referred to as "returned light."

A jaw holding platform 10 is arranged in a position facing the lens tube body 9. The jaw holding platform 10 is provided with a jaw holder 10a and a forehead supporter 10b for stably placing the subject face.

An observation magnification operating knob 11 for changing the observation magnification is arranged on the side surface of the lens tube body 9. Further, an imaging device 13 for imaging the eye E is connected to the lens tube body 9. The imaging device 13 comprises an image sensor. The image sensor is a photoelectric transducer that detects light and outputs electric signals (image signals). The image signals are input into the computer 100. For the image sensor, for example, a CCD (Charge Coupled Device) image sensor or a CMOS (Complementary Metal Oxide Semiconductor) image sensor is used. A mirror 12 for reflecting illumination light beam output from the illumination system 8 towards the eye E is arranged in a lower position of the illumination system 8.

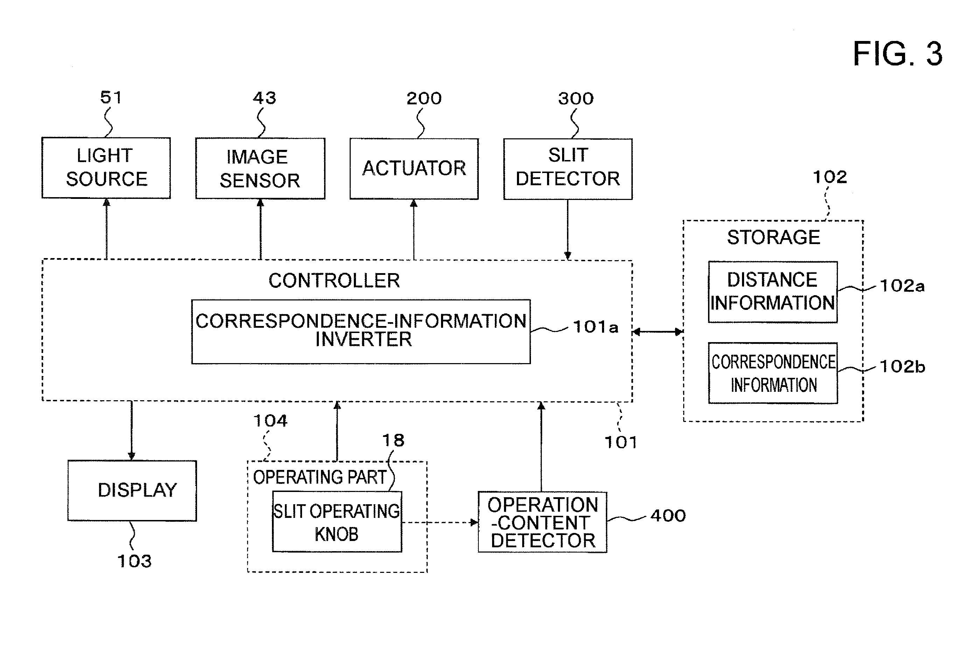

A slit operating knob 18 (see Fig. 3) for changing the slit width is provided, for example, on the side surface of the supporting arm 17 supporting the illumination system 8. The slit operating knob 18 is provided on both the left and right, but is not shown in diagrams. The slit operating knob 18 is configured such that it is capable of rotating around a rotation axis that extends in the left-right direction. The slit operating knob 18 is capable of rotating both clockwise and counterclockwise as seen from the left side. The configuration for changing slit width based on the rotation operation of the slit operating knob 18 is mentioned later. The slit operating knob 18 is an example of a "rotation operation part."

### [Configuration of the optical system]

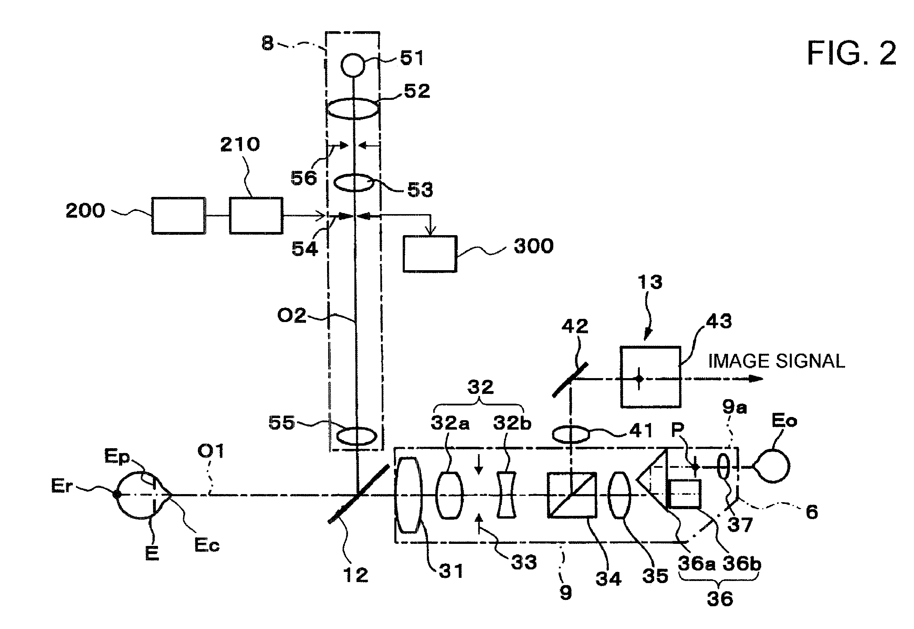

The configuration of the optical system of the slit lamp microscope 1 is described with reference to Fig. 2. The slit lamp microscope 1 comprises the observation system 6 and the illumination system 8.

### [Observation system]

The observation system 6 comprises a left-and-right pair of optical systems. The left and right optical systems have substantially the same configuration. By means of these left and right optical systems, the user is capable of observing the eye E with both eyes. It should be noted that Fig. 2 illustrates only one of the left and right optical systems of the observation system 6. The symbol O1 is the optical axis (observation optical axis) of the observation system 6.

Each of the left and right optical systems of the observation system 6 comprises an objective lens 31, magnification optical system 32, diaphragm 33, relay lens 35, prism 36, and eyepiece lens 37. The beam splitter 34 is provided to only one or both of the left and right optical systems. The eyepiece lens 37 is provided inside the eyepiece 9a. The symbol P indicates the imaging position of the light guided towards the eyepiece lens 37. The symbol Ec indicates the cornea of the eye E, the symbol Ep indicates the iris, and the symbol Er indicates the fundus, respectively. The symbol Eo indicates the user's eye.

The magnification optical system 32 comprises a plurality (for example, two) of magnifying lens 32a and 32b. In this embodiment, a plurality of magnifying lens groups that may be selectively inserted with respect to the optical path of the observation systems 6 are installed. Such magnifying lens groups are configured such that they respectively give different magnification. The magnifying lens group arranged on the optical path of the observation system 6 is used as the magnifying lenses 32a and 32b. Thereby, the magnification (angle of view) of observation images as well as photographed images of the eye E may be changed. The change of the magnification, that is, the replacement of the magnifying lens group arranged on the optical path of the observation system 6 is carried out by operating the observation magnification operating knob 11. Moreover, a configuration is possible wherein the magnification is electrically changed using a switch (not illustrated), etc.

The beam splitter 34 divides the light travelling along the observation optical axis O1 into two. The light that has penetrated the beam splitter 34 is guided to the user's eye Eo via the prism 36, and the eyepiece lens 37. The prism 36 comprises two optical elements 36a and 36b, and parallelly displaces the travelling direction of the light upwards.

Meanwhile, the light that has been reflected by the beam splitter 34 is guided to the image sensor 43 of the imaging device 13 via the relay lens 41 and the mirror 42. The image sensor 43 detects this reflected light to generate image signals.

### [Illumination system]

The illumination system 8 comprises a light source 51, relay lens 52, illumination diaphragm 56, condensing lens 53, slit forming part 54, and condensing lens 55. The symbol 02 indicates the optical axis (illumination optical axis) of the illumination system 8.

The light source 51 outputs illumination light. It should be noted that a plurality of light sources may be provided in the illumination system 8. For example, both a light source outputting a continuous light (halogen lamp, LED, etc.) and a light source outputting a flash light (xenon lamp, LED, etc.) may be provided as the light source 51. Moreover, a light source for cornea observation and a light source for eye-fundus observation may be separately provided.

The slit forming part 54 is used for generating slit light. The slit forming part 54 comprises a pair of slit blades. By changing the interval of these slit blades, the slit width changes.

The slit blades shift by means of an actuator 200 and a driving mechanism 210. The actuator 200 is a device that generates a driving force, and is configured by, for example, a pulse motor. The driving mechanism 210 changes the interval between the slit blades based on the driving force generated by the actuator 200, which corresponds to a "mechanism."

A slit detector 300 is provided in the vicinity of the slit forming part 54. The slit detector 300 detects the event in which the slit blades are positioned in the detection position. The slit detector 300 is, for example, a photo sensor. The photo sensor is a device comprising a light-emitting element and a light-receiving element, detecting the change of intensity of light depending on a detection object (the slit blades) and outputting electric signals.

The illumination diaphragm 56 is configured such that the size of the light-transmitting part thereof may be changed. The illumination diaphragm 56 is particularly effective for eye-fundus observation. For example, the illumination diaphragm 56 is used for reducing reflection of the illumination light by the cornea Ec and/or crystalline lens and adjusting the brightness of the illumination light.

### [Configuration of control system]

The control system of the slit lamp microscope 1 is explained with reference to Fig. 3. The control system of the slit lamp microscope 1 is configured with a controller 101 as the center thereof. Further, Fig. 3 illustrates only the particularly focal components in this embodiment, with other components omitted.

### [Controller]

The controller 101 controls each part of the slit lamp microscope 1. For example, the controller 101 carries out control of the observation system 6, control of the illumination system 8, etc. the control of the observation system 6 includes control of the magnification optical system 32, control of the diaphragm 33, and control of the charge storage time, sensitivity, frame rate, etc. of the image sensor 43. The control of the illumination system 8 includes control of the light source 51, control of the illumination diaphragm 56, and control of the slit forming part 54. It should be noted that the control of the slit forming part 54 is carried out by controlling the actuator 200. If the actuator 200 is a pulse motor, the controller 101 transmits pulse signals to the actuator 200. Moreover, the controller 101 carries out a process of reading data stored in the storage 102 and a process of writing data into the storage 102.

Electric signals from the slit detector 300 are input into the controller 101. Thereby, the controller 101 recognizes that the slit blades are positioned in the detection position. It should be noted that the detection outcome by the slit detector 300 is used for positioning the pair of slit blades of the slit forming part 54 in the standard slit position (mentioned later). The standard slit position is, for example, the position at which the pair of slit blades contact each other, that is, the position of the pair of slit blades in which the slit width is substantially zero.

Electric signals from the operation-content detector 400 are input into the controller 101. The operation-content detector 400 detects the operation content with respect to the operation part 104 for changing the slit width. In this embodiment, this operation content is the displacement of the operation part 104 with respect to a predetermined standard operation position. Specifically, in such an embodiment, the slit width is changed by rotatingly operating the slit operating knob 18, and the operation-content detector 400 detects the rotation position of the slit operating knob 18 with respect to the predetermined standard operation position.

An example of a configuration of the operation-content detector 400 is explained. The operation-content detector 400 comprises an encoder that detects the rotation position of the slit operating knob 18. This encoder comprises, for example, a light shading plate integrally rotating together with the slit operating knob 18, and a photo sensor with a light-emitting element as well as a light-receiving element arranged facing each other in positions sandwiching this light shading plate. The light shading plate is formed with a plurality of light-transmitting parts (slits) arranged at equal intervals. The light-emitting element is continuously turned on. Each time the light-transmitting part passes through the position between the light-emitting element and the light-receiving element along with the rotation of the slit operating knob 18, the light-receiving element transmits an electric signal to the controller 101. The controller 101 counts the number of the electric signals input from the light-receiving element, thereby recognizing the rotation position of the slit operating knob 18.

The correspondence-information inverter 101a inversely processes the correspondence information 102b mentioned later. The processes carried out by the correspondence-information inverter 101a are mentioned later.

The controller 101 comprises hardware such as a microprocessor, RAM, ROM, hard disk drive, etc. A control program is stored in advance in this hard disk drive. Operations of the controller 101 are realized by cooperation of the control program and the aforementioned hardware.

The controller 101 is arranged in the main body (for example, in base 4) of the slit lamp microscope 1 and/or the computer 100.

### [Storage]

A variety of information is stored in the storage 102. Specifically, distance information 102a and correspondence information 102b are stored in advance in the storage 102.

The distance information 102a indicates the distance between the slit blade position when the slit blades of the slit forming part 54 are closed and the detection position mentioned above. This distance does not need to be a special distance and should be a quantity equivalent to the distance. For example, if the actuator 200 is a pulse motor, the distance information 102a may be the number of pulses of the pulse signal transmitted from the controller 101 to the pulse motor.

The distance information 102a may be generated, for example, by the following processes: causing the slit detector 300 to detect the event in which the slit blades are located in the detection position; inputting pulse signals into the pulse motor from this state until the slit blade closes; counting the number of pulses of the input pulse signals. The distance information 102a is made, for example, prior to product shipment of the slit lamp microscope 1. Moreover, the distance information may be revised after commencing use of the slit lamp microscope 1.

The correspondence information 102b is the information in which the operation content with respect to the operation part 104 and the operation amount of the actuator 200 are associated with each other. In this embodiment, the rotation position of the slit operating knob 18 is used as the operation content of the operation part 104, while the number of pulses of the pulse signals transmitted to the actuator 200 is used as the operation amount of the actuator 200. It should be noted that, in this embodiment, operations of changing the slit width is carried out after arranging the pair of slit blades of the slit forming part 54 in the standard slit position; therefore, the number of pulses may be substantially identified with the position of the pair of slit blades (that is, the slit width). Hereinafter, an example of the correspondence information 102b is explained.

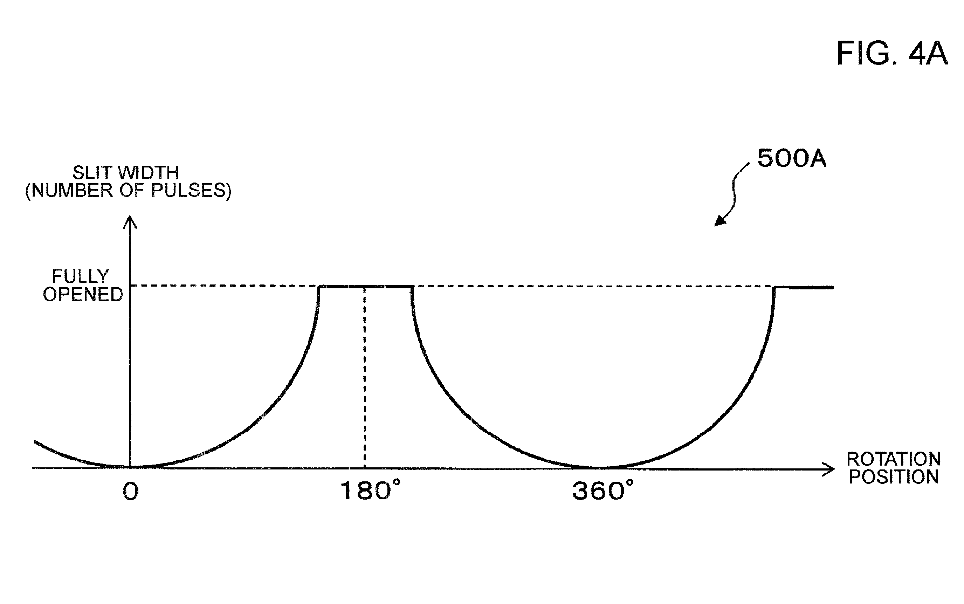

A first example of the correspondence information 102b is illustrated in Fig. 4A. Graph 500A illustrates the correspondence between the rotation position of the slit operating knob 18 and the slit width (number of pulses) in the first example of the correspondence information 102b. The horizontal axis of the graph 500A indicates the rotation position, while the vertical axis indicates the slit width. The rotation position is defined in two directions, the positive direction and the negative direction, with respect to the standard operation position of 0 degrees. The slit width is defined between the slit width 0 corresponding to the standard slit position and the maximum slit width in which the slit is fully opened.

Moreover, the graph 500A is defined such that it is symmetrical repetition is made in the positive direction and negative direction with the standard operation position of 0 degrees at the center at a cycle of 360 degrees. That is, the graph 500A is regarded as a periodic function that is obtained by extending the function defined in the range of rotation position 0 degrees to 360 degrees to arbitrary range of the rotation position. Accordingly, the rotation operation of the slit operating knob 18 is recognized by the controller 101 with modulo 360 degrees (that is, a difference by 360 degrees is ignored). In this example, it is possible to restrict the domain of definition of the correspondence information 102b within a single period (that is, a range of rotation position 0 degrees to 360 degrees) and store this in the storage 102.

Within the range of rotation position 0 degrees to 360 degrees, the graph 500A is identified as a function symmetric in the positive direction and negative direction, with the rotation position of 180 as the center. Specifically, the graph 500A is monotonically increasing in the specific range (positions in front of 180 degrees) from the standard operation position 0 degrees towards the positive direction (first direction). In this monotonically increasing range (first range), the inclination of the graph 500A smoothly increases from the standard operation position of 0 degrees towards the positive direction. That is, in this monotonically increasing range of the graph 500A, the amount of change of the slit width (operation amount) with respect to the unit amount of change of the rotation position monotonically increases. Such a shape of the graph 500A indicates that fine adjustment of the slit width is possible when the slit width is narrow, while rough adjustment is possible when the slit width is wide.

Moreover, in the range from the end position of the positive direction of the above monotonically increasing range to the rotation position exceeding the rotation position of 180 degrees, the inclination of the graph 500A is 0. That is, in this flat range (third range), the slit width does not change (that is, the operation amount is 0) even when the rotation position is changed. This corresponds to the play with respect to the rotation operation of the slit operating knob 18. It should be noted that it is possible to provide similar play in the range including the standard operation position of 0 degrees.

Further, in the range from the end position of the positive direction in the above play range to the rotation position of 360 degrees, the graph 500A is monotonically decreasing. In this monotonically decreasing range (second range), the inclination of the graph 500A smoothly decreases from the end position of the positive direction in the play range towards the positive direction. That is, in this monotonically decreasing range of the graph 500A, the amount of change of the slit width (operation amount) with respect to the unit amount of change of the rotation position monotonically decreases. Such a shape of the graph 500A, in the same way as the above, indicates that fine adjustment of the slit width is possible when the slit width is narrow, while rough adjustment is possible when the slit width is wide.

When the first example as above is applied, the user is capable of rotating the slit operating knob 18 in the desired direction. In any rotation direction, increase and decrease of the slit width are repeated along with the increase in the amount of rotation of the slit operating knob 18. Moreover, as mentioned above, fine adjustment of the slit width is possible when the slit width is narrow, while rough adjustment is possible when the slit width is wide.

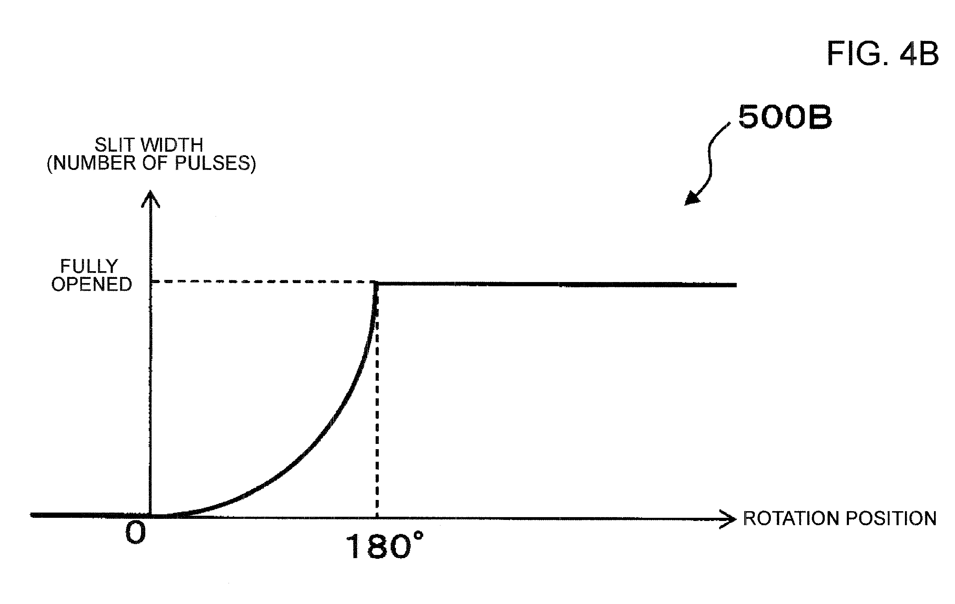

A second example of the correspondence information 102b is illustrated in Fig. 4B. The graph 500B illustrates the correspondence between the rotation position of the slit operating knob 18 and the slit width (number of pulses) in the second example of the correspondence information 102b. The horizontal axis of the graph 500B indicates the rotation position while the vertical axis indicates the slit width. The rotation position is defined in the positive direction alone with respect to the standard operation position of 0 degrees, which differs from the first example. The slit width is defined between the slit width 0 corresponding to the standard slit position and the maximum slit width in which the slit is fully opened.

The graph 500B is monotonically increasing within the range from the standard operation position of 0 degrees to the rotation position of 180 degrees. This monotonically increasing range (first range) is the same as the first example, excluding the difference in the end position of the positive direction. Moreover, within the range that is on the positive direction side of the rotation position of 180 degrees, the inclination of the graph 500B is 0. Similarly, the inclination of the graph 500B is also 0 within the range that is on the negative direction side of the standard operation position of 0 degrees. There is no substantial difference between these flat ranges and that in the first example.

When the second example as above is applied, the user rotates the slit operating knob 18 in the rotation direction corresponding to the positive direction when increasing the slit width. Conversely, when decreasing the slit width, the user rotates the slit operating knob 18 in the rotation direction corresponding to the negative direction. Moreover, when the slit width is at its maximum, even when the slit operating knob 18 is rotated in the rotation direction corresponding to the positive direction, the slit width does not change. Moreover, in the same manner as the first example, fine adjustment is possible when the slit width is narrow, while rough adjustment is possible when the slit width is wide.

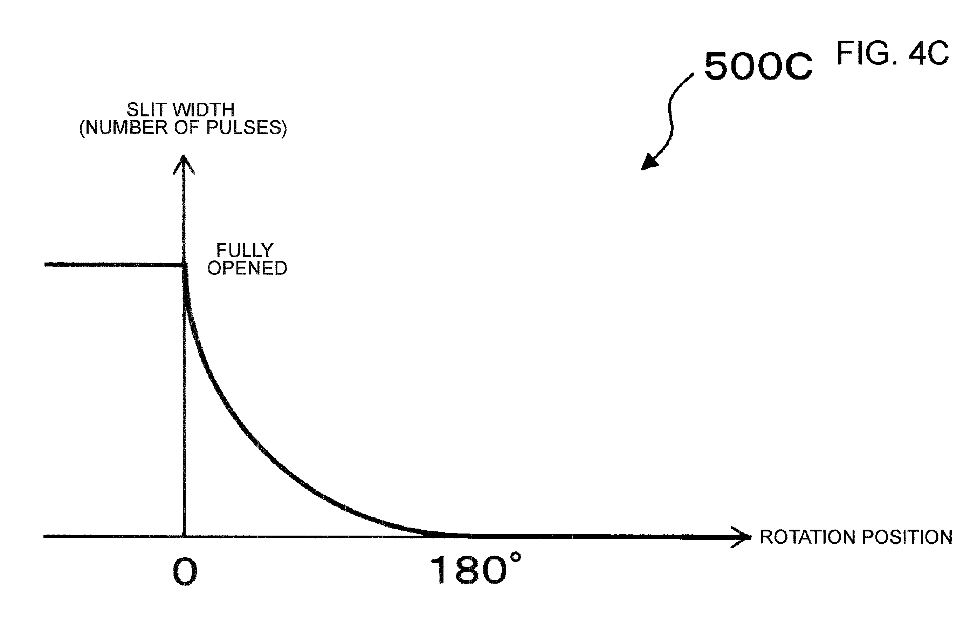

The third example of the correspondence information 102b is illustrated in Fig. 4C. The graph 500C illustrates the correspondence between the rotation position of the slit operating knob 18 and the slit width (number of pulses) in the third example of the correspondence information 102b. The horizontal axis of the graph 500C indicates the rotation position while the vertical axis indicates the slit width. The rotation position is, in the same manner as the first example, defined in two directions, the positive direction and negative direction with respect to the standard operation position of 0 degrees. The slit width is defined between the slit width 0 corresponding to the standard slit position and the maximum slit width in which the slit is fully opened.

In the graph 500C, the maximum slit width is associated with the standard operation position of 0 degrees. The graph 500C is constant (maximum slit width) in the negative direction than the standard operation position of 0 degrees. Moreover, within the range from the standard operation position of 0 degrees to the rotation position of 180 degrees, the graph 500C is monotonically decreasing. In this monotonically decreasing range (first range), as in the first example, the inclination of the graph 500C smoothly decreases from the standard operation position of 0 degrees towards the positive direction.

In the case of applying the third example as above, when decreasing the slit width, the user rotates the slit operating knob 18 in the rotation direction corresponding to the positive direction. In contrast, when increasing the slit width, the user rotates the slit operating knob 18 in the rotation direction corresponding to the negative direction. Moreover, when the slit width is at its maximum, even when the slit operating knob 18 is rotated in the rotation direction corresponding to the negative direction, the slit width does not change. Moreover, in the same manner as the first example, fine adjustment is possible when the slit width is narrow, while rough adjustment is possible when the slit width is wide.

The first to third examples explained as above are merely typical examples of the correspondence information 102b; therefore, it is possible to apply correspondence information 102b other than that mentioned above. Moreover, it is possible to store a plurality of correspondence information 102b in the storage 102. In this case, any selected correspondence information 102b is used. The selection of the correspondence information 102b may be manually carried out by the user or automatically selected by the controller 101. Manual selection may be carried out by, for example, the controller 101 displaying the choices of correspondence information 102b on the display 103, then the user selecting the desired information from among these using the operation part 104. In the case of automatic selection, for example, information indicating the correspondence information 102b applied to the patient under concern (the eye E under concern) in the past is stored in the storage 102 together with the identification information (patient identification information and eye identification information). Subsequently, the correspondence information 102b associated with the input identification information is read from the storage 102 and then applied by the controller 101.

### [Display]

The display 103 is controlled by the controller 101 to display a variety of information. The display 103 comprises any kinds of display device, for example, a flat panel display such as LCD, etc., or a CRT display. The display 103 may be provided on the main body of the slit lamp microscope 1 or on the computer 100.

### [Operating part]

The operating part 104 comprises an operation device and/or an input device. The operating part 104 comprises buttons and switches provided on the main body (for example, an operation handle 5, etc.) and a mouse, keyboard, etc. of the computer 100. Moreover, it is possible to use any operation devices and input devices such as a trackball, dedicated operation panel, switch, button, dial, etc.

The operation part 104 comprises the slit operating knob 18. As mentioned above, the operation content of the slit operating knob 18 is detected by the operation-content detector 400 and the detection outcome thereof is input into the controller 101.

In Fig. 3, the display 103 and the operating part 104 are separately illustrated; however, these may be integrally configured. As a detailed example thereof, a touch panel LCD may be used.

### [Control of slit width]

The control upon changing the slit width is explained. When the user operates the slit operating knob 18, the operation-content detector 400 detects the operation content (rotation position) thereof. The operation-content detector 400 transmits the detection results to the controller 101. The controller 101 obtains the slit width (number of pulses) associated with the detected rotation position based on the correspondence information 102b. Then, the controller 101 generates pulse signals with the obtained number of pulses and transmits these to the actuator 200 (pulse motor). The actuator 200 generates the driving force in accordance with the number of pulses of these pulse signals to move the pair of slit blades of the slit forming part 54, thereby realizing the targeted slit width.

### [Inversion of correspondence information]

The processes carried out by the correspondence-information inverter 101 a are explained. The correspondence-information inverter 101a inverts the correspondence between the rotation direction of the slit operating knob 18 and the slit width (number of pulses, operation amount of the actuator 200) in the correspondence information 102b. This inversion process is carried out following, for example, instructions by the user. Moreover, the inversion processes may be carried out based on the information indicating the implementation of the inversion in the past examination for the patient under concern (the eye E under concern).

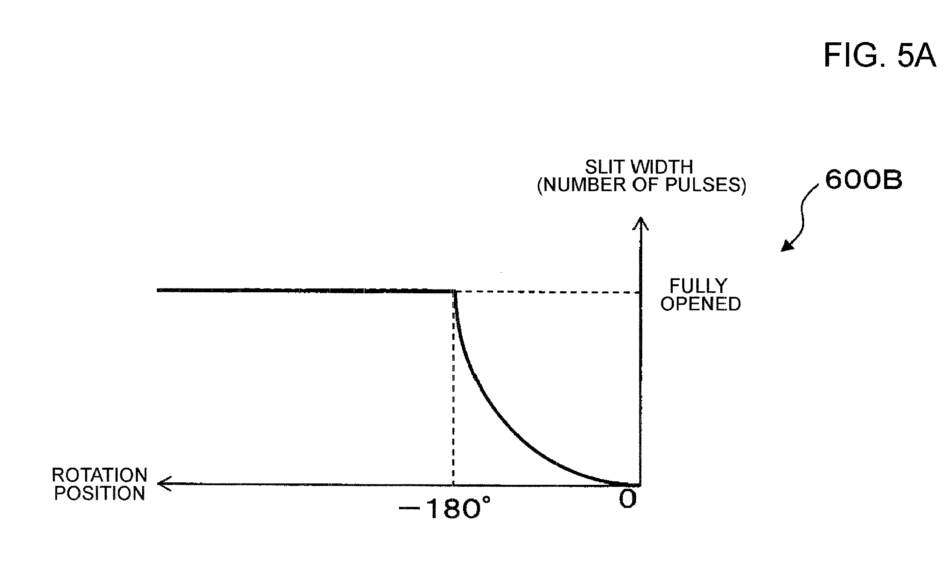

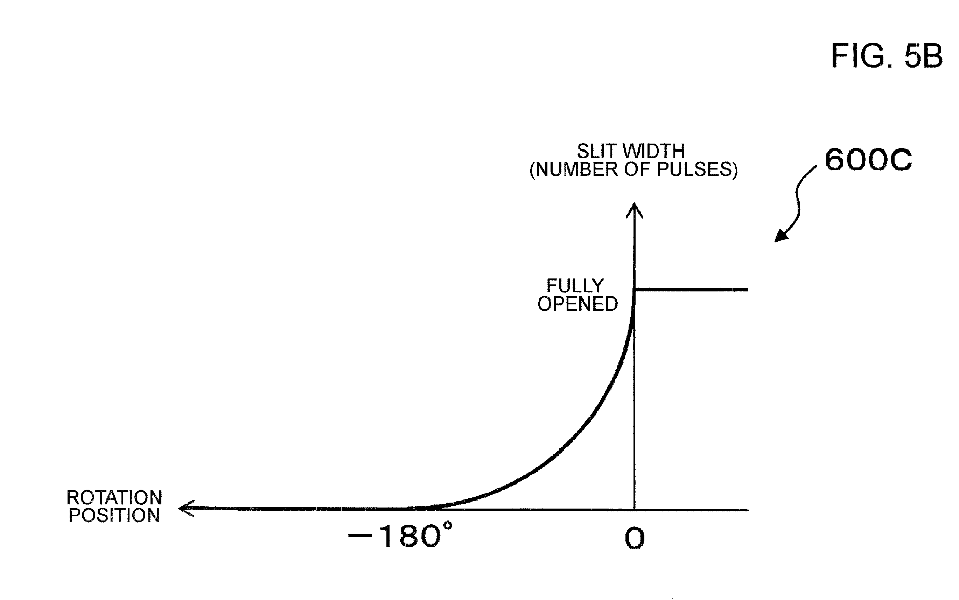

A specific example of inversion processing is explained. The correspondence-information inverter 101a inverts the correspondence information 102b with the standard operation position at the center. When the graph 500B illustrated in Fig. 4B is inverted, the graph 600B illustrated in Fig. 5A is obtained. Moreover, when the graph 500C illustrated in Fig. 4C is inverted, the graph 600C illustrated in Fig. 5B is obtained. It should be noted that the graph 500A illustrated in Fig. 4A is symmetric in the horizontal- axis direction with respect to the standard operation position of 0 degrees; therefore, the shape does not change even when inverted.

In the event the correspondence information 102b is inverted, the controller 101 changes the slit width using the correspondence information 102b obtained upon inversion processing.

### [Operation]

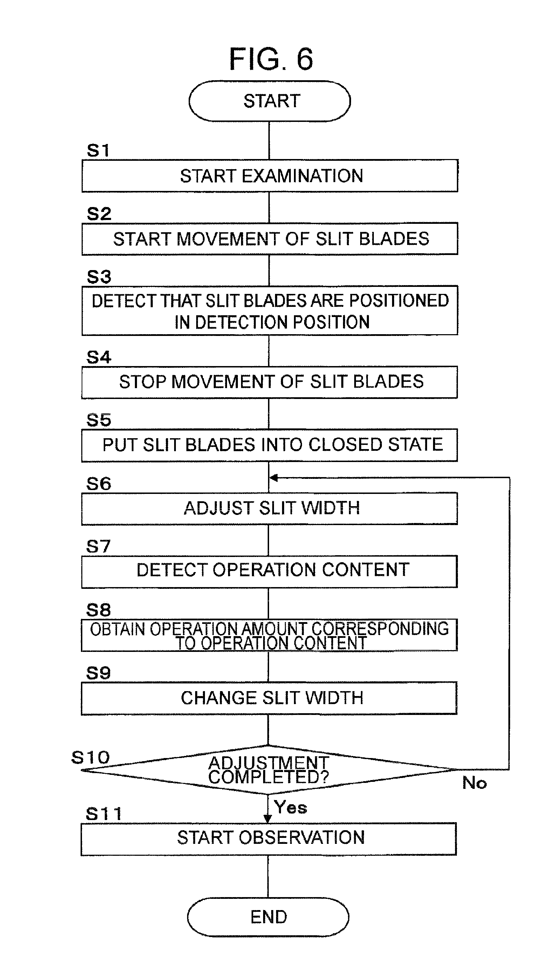

The operation of the slit lamp microscope 1 is explained. An example of the operation of the slit lamp microscope 1 is illustrated in Fig. 6.

In the example below, Steps S1 to S5 are carried out in response to the commencement of an examination; however, it is possible to carry out these processes at any timing, such as, power activation, inputting patient identification information, changing the eye E, etc. Further, it is assumed that the distance information 102a and the correspondence information 102b are already stored. Moreover, in the case in which a plurality of correspondence information 102b is stored, selection of the correspondence information 102b to be used is carried out at any timing. Similarly, the inversion of the correspondence information 102b is carried out at any timing.

### (S1 : Start examination)

Arrange the eye E in the examination position facing the optical system of the slit lamp microscope 1. The user carries out specific operations to instruct commencement of examination.

### (S2: Start movement of slit blades)

The controller 101 controls the actuator 200 and moves the slit blades.

### (S3: Detect that slit blades are positioned in detection position)

The controller 101 detects that the slit blades are positioned in the detection position based on electric signals from the slit detector 300.

### (S4: Stop movement of slit blades)

Upon detecting that the slit blades are positioned in the detection position, the controller 101 controls the actuator 200 and stops the movement of the slit blades.

### (S5: Put slit blades into closed state)

The controller 101 controls the actuator 200 based on the distance information 102a stored in the storage 102 to move the slit blades, thereby putting the slit blades into a closed state. This process is carried out by, for example, generating pulse signals with the number of pulses indicated in the distance information 102a and then transmitting the pulse signals to the actuator 200 (pulse motor).

For example, at this stage, the light source 51 is lit. Moreover, the user adjusts the positions of the observation systems 6 and the illumination systems 8.

### (S6: Adjust slit width)

The user operates the slit operating knob 18 while observing the eye E, thereby adjusting the slit width.

### (S7: Detect operation content)

The operation-content detector 400 detects the operation content of the rotation operation (rotation position) carried out with respect to the slit operating knob 18. The operation-content detector 400 inputs the detection result into the controller 101.

### (S8: Obtain operation amount corresponding to operation content)

The controller 101 obtains the operation amount (slit width, number of pulses) of the actuator 200 corresponding to the operation content detected at Step S7 based on the correspondence information 102b.

### (S9: Change slit width)

The controller 101 controls the actuator 200 based on the operation amount obtained in Step S8, thereby changing the interval between the pair of slit blades of the slit forming part 54 by the distance corresponding to the operation content detected at Step S7. This process is carried out by, for example, transmitting the pulse signals with the number of pulses obtained in Step S8 to the pulse motor (actuator 200).

### (S10: Adjustment completed?)

The processes of Step S6 to Step S9 are repeated until the slit width comes to the desired state (S10: No).

### (S11: Start observation)

Meanwhile, once slit width adjustment is completed (S10: Yes), observation of the eye E may be commenced using the slit light. This concludes the explanation of the operation example.

### [Effect]

The effect of the slit lamp microscope 1 is explained.

The slit lamp microscope 1 comprises the light source 51, the pair of slit blades (the slit forming part 54), the actuator 200, the mechanism, the operation part 104, the storage 102, the operation-content detector 400, and the controller 101. The light source 51 emits illumination light. The pair of slit blades generates slit light from the illumination light. The actuator 200 generates a driving force. The mechanism changes the interval between the pair of slit blades based on the driving force. The operation part 104 is used for adjusting the interval between the pair of slit blades (the slit width). The storage 102 stores in advance the correspondence information 102b in which the operation content with respect to the operation part 104 and the operation amount of the actuator 200 are associated each other. The operation-content detector 400 detects the operation content with respect to the operation part 104. The controller 101 obtains the operation amount associated with the detected operation content based on the correspondence information 102b and controls the actuator 200 based on the detected operation amount, thereby changing the slit width by the distance corresponding to this operation content.

According to such a slit lamp microscope 1, the slit width may be electrically controlled based on the correspondence information 102b; thereby allowing easy adjustment of the slit width with high precision to be realized.

The operation content may be the displacement of the operation part 104 with respect to the predetermined standard operation position. In this case, the correspondence information 102b associating the displacement of the operation part 104 and the operation amount of the actuator 200 may be used so as to change the operation amount in at least a part of a variable range of the displacement of the operation part 104.

In the example illustrated in Fig. 4A, the variable range of the displacement (rotation position) of the operation part 104 is -∞ to +∞. Moreover, the partial ranges in which the operation amount changes are the monotonically increasing range from 0 degrees to the front position of 180 degrees, the monotonically decreasing range from a position exceeding 180 degrees to 360 degrees, and any ranges congruent to these ranges with modulo 360 degrees.

In the example illustrated in Fig. 4B, the variable range of the displacement (rotation position) of the operation part 104 is -∞ to +∞. Moreover, the partial range in which the operation amount changes is the monotonically increasing range from 0 degrees to 180 degrees.

In the example illustrated in Fig. 4C, the variable range of the displacement (rotation position) of the operation part 104 is -∞ to +∞. Moreover, the partial range in which the operation amount changes is the monotonically decreasing range from 0 degrees to 180 degrees.

Moreover, the correspondence information 102b may be information that associates the standard operation position of the operation part 104 and the standard slit position of the pair of slit blades in which the slit width is substantially zero. Further, the slit lamp microscope 1 may comprise the slit detector 300 that detects a state in which the pair of slit blades are positioned in the specific detection position. In this case, it is possible to configure that the distance information 102a indicating the distance between the standard slit position and the detection position is stored in the storage 102, and that when detecting that the slit blades are positioned in the detection position the controller 101 controls the actuator 200 based on the distance information 102a, thereby arranging the pair of slit blades in the standard slit position. By means of adopting such a configuration, the standard position of the slit width adjustment may be set, making it possible to associate the amount of change in slit width (number of pulses) and the slit width itself. Thereby, easy adjustment of the slit width with high precision may be realized.

The operation part 104 may comprise the slit operating knob 18 (rotation operation part) rotatable in the first direction and the second direction which is the opposite direction to the first direction. Here, the combination of the first direction and the second direction corresponds to the combination of the positive direction and the negative direction mentioned above. It should be noted that the positive direction and the negative direction are arbitrarily set in advance.

In this case, the operation content with respect to the slit operating knob 18 includes the rotation position of the slit operating knob 18 with respect to the standard operation position. Here, the rotation position is obtained by considering both the positive direction and the negative direction (that is, the positive rotation and the negative rotation are arbitrarily defined). Further, it is possible to use the correspondence information 102b in which, in the first range from the standard operation position towards the first direction, the rotation position and the operation amount are associated with each other such that the amount of change of the operation amount of the actuator 200 with respect to the unit amount of change of the rotation position of the slit operating knob 18 monotonically varies. It should be noted that the first range corresponds to the monotonically increasing ranges in the examples illustrated in Fig. 4A and Fig. 4B, and corresponds to the monotonically decreasing range in the example illustrated in Fig. 4C. Further, in the case in which the first direction in the first range is the rotation direction for increasing the slit width, the monotonic variation mentioned above becomes a monotonic increase. Conversely, in the case of the first direction in the first range being the rotation direction for decreasing the slit width, the monotonic variation mentioned above becomes a monotonic decrease. In this manner, by means of providing the first range in which the amount of change of the operation amount monotonically varies with respect to the unit amount of change of the rotation position, fine adjustment as well as rough adjustment of the slit width becomes possible. Accordingly, easy adjustment of the slit width with high precision may be realized.

Moreover, in the second range differing from the first range, it is possible to use the correspondence information 102b associating the rotation position and the operation amount such that the amount of change of the operation amount of the actuator 200 with respect to the unit amount of change of the rotation position of the slit operating knob 18 in the first direction becomes a reverse monotonic variation compared to the monotonic variation in the first range. The second range corresponds to the monotonically decreasing range in the example illustrated in Fig. 4A. According to this configuration, the slit width may be increased and decreased while rotating the slit operating knob 18 in the same direction. Further, fine adjustment and rough adjustment of the slit width may be easily carried out while rotating the slit operating knob 18 in the same direction. Accordingly, easy adjustment of the slit width with high precision may be realized.

Moreover, it is possible to use the correspondence information 102b associating the rotation position and the operation amount so as to include the third range associated with an operation amount of zero with respect to the change of rotation position of the slit operating knob 18. The third range corresponds to the flat range in the example illustrated in Fig. 4A. According to this configuration, it is possible to set the play in the rotation operation of the slit operating knob 18, thereby encouraging enhanced operability. This contributes to the high precision and simplification of the slit width adjustment.

Moreover, the controller 101 may comprise the correspondence-information inverter 101a that inverts the correspondence between the rotation direction (the first direction and the second direction) of the slit operating knob 18 and the operation amount of the actuator 200 in the correspondence information 102b. By means of inverting the correspondence information 102b in this manner, variations of the correspondence information 102b may be increased and enhanced operability as well as easy adjustment of the slit width with high precision may be realized.

It is possible to store a plurality of correspondence information 102b in the storage 102 in advance. In this case, the controller 101 selectively uses the plurality of correspondence information 101b, thereby obtaining the operation amount associated with the operation content detected by the operation-content detector 400. By means of enabling selective usage of the plurality of correspondence information 102b in this manner, variations of the correspondence information 102b may be increased and enhanced operability as well as easy adjustment of the slit width with high precision may be realized.

The configurations explained above are merely examples for implementing the present invention. One attempting to implement the present invention is allowed any deformation, addition, omission, replacement within the scope of the present invention.

### [Explanation of Symbols]

- 1: slit lamp microscope
- 6: observation system
- 8: illumination system
- 18: slit operating knob
- 43: image sensor
- 100: computer
- 101: controller
- 101a: correspondence-information inverter
- 102: storage
- 102a: distance information
- 102b: correspondence information
- 103: display
- 104: operating part
- 200: actuator
- 300: slit detector
- 400: operation-content detector
- 500A, 500B, 500C: graph
- 600B, 600C: inverted graph
- E: eye

## Claims

1. A slit lamp microscope, comprising:
a light source configured to emit illumination light;
a pair of slit blades configured to generate slit light from the illumination light;
an actuator configured to generate a driving force;
a mechanism configured to change the interval between the slit blades based on the driving force;
an operation part;
a storage configured to store in advance correspondence information in which the operation content with respect to the operation part and the operation amount of the actuator are associated;
an operation-content detector configured to detect an operation content with respect to the operation part; and
a controller configured to obtain the operation amount associated with the detected operation content based on the correspondence information and control the actuator based on the obtained operation amount, thereby changing the interval of the slit blades by a distance corresponding to this operation content.

2. The slit lamp microscope according to Claim 1, wherein
the operation content includes the displacement of the operation part with respect to a predetermined standard operation position, and
the displacement and the operation amount are associated in the correspondence information such that the operation amount changes in at least a part of a variable range of the displacement.

3. The slit lamp microscope according to Claim 2, wherein
the standard operation position and the standard slit position of the slit blades such that the interval is substantially zero are associated in the correspondence information.

4. The slit lamp microscope according to Claim 3, comprising
a slit detector configured to detect the state in which the slit blades are positioned in a predetermined detection position, wherein
the storage stores in advance distance information indicating the distance between the standard slit position and the detection position,
and
when it is detected that the slit blades are positioned in the detection position, the controller controls the actuator based on the distance information, thereby positioning the slit blades in the standard slit position.

5. The slit lamp microscope according to any one from among Claims 2 to 4, wherein
the operation part comprises a rotation operation part capable of rotating in a first direction and a second direction which is the opposite direction thereof,
the operation content includes the rotation position of the rotation operation part with respect to the standard operation position,
and
the rotation position and the operation amount are associated in the correspondence information such that the amount of change of the operation amount with respect to the unit amount of change of the rotation position monotonically varies within a first range from the standard operation position towards the first direction.

6. The slit lamp microscope according to Claim 5, wherein,
in the first range, the first direction is the rotation direction of the rotation operation part for increasing the interval of the slit blades, and
the monotonic variation is a monotonic increase.

7. The slit lamp microscope according to Claim 5, wherein,
in the first range, the first direction is the rotation direction of the rotation operation part for decreasing the interval of the slit blades, and
the monotonic variation is a monotonic decrease.

8. The slit lamp microscope according any one from among Claims 5 to 7, wherein
the rotation position and the operation amount are associated in the correspondence information such that, in a second range differing from the first range, the amount of change of the operation amount with respect to the unit amount of change of the rotation position in the first direction monotonically varies inversely with the monotonic variation.

9. The slit lamp microscope according any one from among Claims 5 to 8, wherein
the rotation position and the operation amount are associated in the correspondence information such that it includes a third range in which zero is associated as the operation amount with respect to the change of the rotation position.

10. The slit lamp microscope according any one from among Claims 5 to 9, wherein
the controller comprises a correspondence-information inverter configured to invert the correspondence between the rotation direction of the rotation operation part and the operation amount in the correspondence information.

11. The slit lamp microscope according any one from among Claims 1 to 10, wherein
the storage stores in advance a plurality of the correspondence information, and
the controller selectively uses the plurality of correspondence information to obtain the operation amount associated with the operation content detected by the operation-content detector.

12. The slit lamp microscope according any one from among Claims 1 to 11, wherein
the actuator is a pulse motor, and
the operation amount indicates the number of pulses of pulse signals transmitted from the controller to the pulse motor.
